# FASCICULE DE BREVET EUROPEEN

(11) **EP 0 900 100 B1**
(45) Date de publication et mention de la délivrance du brevet: **02.07.2003**
(21) Numéro de dépôt: 97919495.8
(22) Date de dépôt: 09.04.1997
(51) Int. Cl.: A61M 15/00

(54) **INHALATEUR A POUDRE ET A AIR COMPRIME**
PULVERINHALATOR MIT DRUCKLUFTERZEUGUNG
COMPRESSED AIR POWDER INHALER

(30) Priorité: 10.04.1996 FR 9604446
(43) Date de publication de la demande: 10.03.1999
(73) Titulaire: PIERRE FABRE MEDICAMENT, 92100 Boulogne-Billancourt (FR)
(72) Inventeur: MARCON, Christian, F-94000 Créteil (FR)
(74) Mandataire: Ahner, Francis
(86) Numéro de dépôt international: FR9700624
(87) Numéro de publication internationale: WO97037707

(56) Documents cités:
- EP-A- 0 490 797
- US-A- 5 287 850
- US-A- 5 347 999
- US-A- 5 388 572

## Description

L'invention concerne les inhalateurs à poudre.

On connaît d'après le document EP-0 549 605 B₁ un inhalateur à poudre, notamment pour traitement médicamenteux, comportant une chambre d'alimentation en air communiquant avec un conduit débouchant dans une embouchure d'aspiration. Des moyens sont prévus pour disposer dans le conduit avant chaque utilisation une dose de poudre à inhaler. L'air est envoyé dans le conduit, emporte la poudre, franchit l'embouchure et aboutit dans les poumons de l'utilisateur. L'envoi de l'air facilite l'administration de la poudre dans les poumons sans exiger de l'utilisateur un effort d'inspiration qu'il n'est parfois pas capable de fournir. Pour que la réussite du traitement soit optimale, toute la dose de poudre doit être acheminée profondément dans les poumons. Or cela n'est possible que si l'air est envoyé au moment exact où l'utilisateur inspire. A cette fin, l'inhalateur comporte un tiroir mobile à coulissement dans le conduit d'inhalation et sensible à une aspiration dans l'embouchure. Ce tiroir est relié à un piston de la chambre qu'il verrouille en position à l'encontre d'un ressort de rappel comprimé. Lorsque le patient aspire, le déplacement du tiroir libère le piston dont le mouvement génère le flux d'air dans le conduit pour entraîner la poudre. Ainsi, l'air est envoyé en synchronisation avec l'inspiration du patient. Cependant, le mécanisme de synchronisation de l'envoi de l'air avec l'inspiration, s'étendant depuis le conduit jusqu'au piston, est relativement encombrant et compliqué.

Le document US-5 388 572 présente un inhalateur à poudre tel que défini dans le préambule de la revendication 1, comportant une chambre d'alimentation en air et de compression d'air, un conduit communiquant avec la chambre par un orifice et débouchant dans une embouchure d'inhalation, des moyens pour disposer une dose de poudre dans le conduit, et un détecteur d'une aspiration créée dans l'embouchure, l'inhalateur comportant en outre un obturateur mobile agencé pour pouvoir obturer l'orifice, des moyens pour éloigner l'obturateur par rapport à l'orifice, ces moyens étant commandés par le détecteur, et pour solliciter l'obturateur dans le sens de son éloignement de l'orifice lorsqu'il obture l'orifice. Sous l'effet d'un ressort, un piston, libéré par le détecteur, compresse l'air de la chambre et vient déplacer l'obturateur.

Un but de l'invention est de fournir un inhalateur à poudre et à air comprimé comportant un mécanisme de synchronisation, qui soit de structure simple et peu encombrante et permette un envoi soudain de l'air.

En vue de la réalisation de ce but, on prévoit selon l'invention un inhalateur à poudre tel que revendiqué dans la revendication indépendante 1.

L'envoi de l'air comprimé est commandé par le déplacement de l'obturateur. La libération de l'orifice permet l'envoi soudain de l'air de sorte que toute la poudre est administrée durant l'aspiration. Le mécanisme de commande synchronisée de l'obturateur est de structure simple et peu encombrante.

D'autres caractéristiques et avantages de l'invention apparaîtront encore dans la description suivante d'un mode de réalisation donné à titre d'exemple non limitatif. Aux dessins annexés:
- la figure 1 est une vue en coupe axiale de face d'un inhalateur selon l'invention au repos;
- la figure 2 est une vue en coupe axiale de gauche de l'inhalateur de la figure 1;
- la figure 3 est une vue partielle analogue à la figure 2 montrant le levier en position levée;
- la figure 4 est une vue partielle à échelle agrandie analogue à la figure 2 montrant le mécanisme à volet lorsqu'une inspiration est créée dans l'embouchure;
- la figure 5 est une vue de face de la crémaillère;
- la figure 6 est une vue à échelle agrandie du détail D de la figure 2;
- la figure 7 est une vue à échelle agrandie du détail E de la figure 4;
- la figure 8 est une vue en perspective de l'obturateur et du rochet;
- la figure 9 est une vue en perspective du porte-piston;
- la figure 10 est une vue en coupe axiale du porte-piston et du plateau;
- la figure 11 est une vue en coupe transversale selon le plan XI-XI de la figure 10;
- la figure 12 est une vue à échelle agrandie du détail F de la figure 2;
- la figure 13 est une vue en perspective de la réserve de poudre;
- la figure 14 est une vue de dessous de l'embase;
- la figure 15 est une vue en coupe suivant le plan XV-XV de la figure 4; et
- la figure 16 est une vue en coupe suivant le plan XVI-XVI de la figure 2.

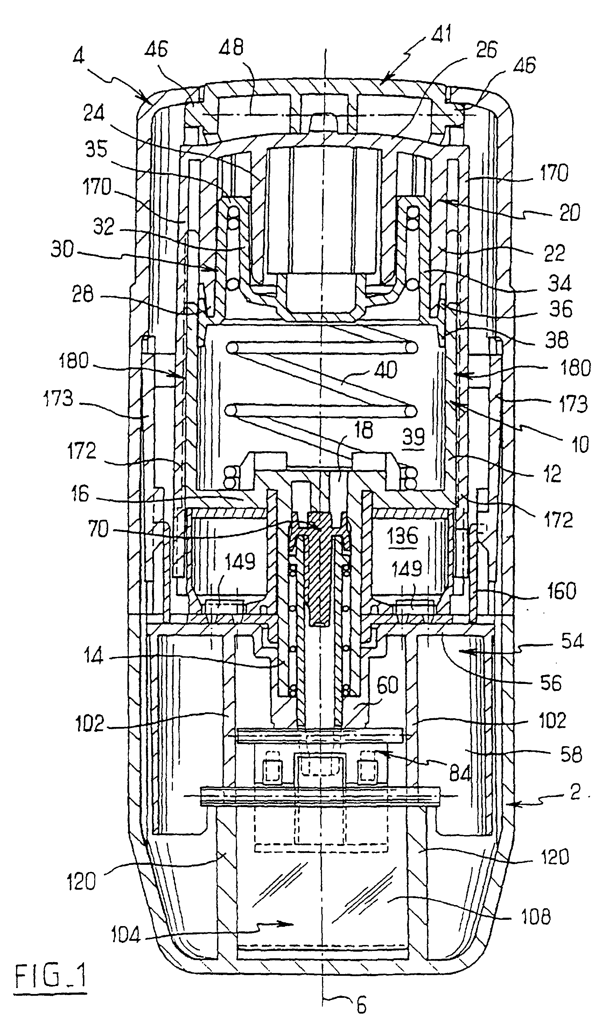

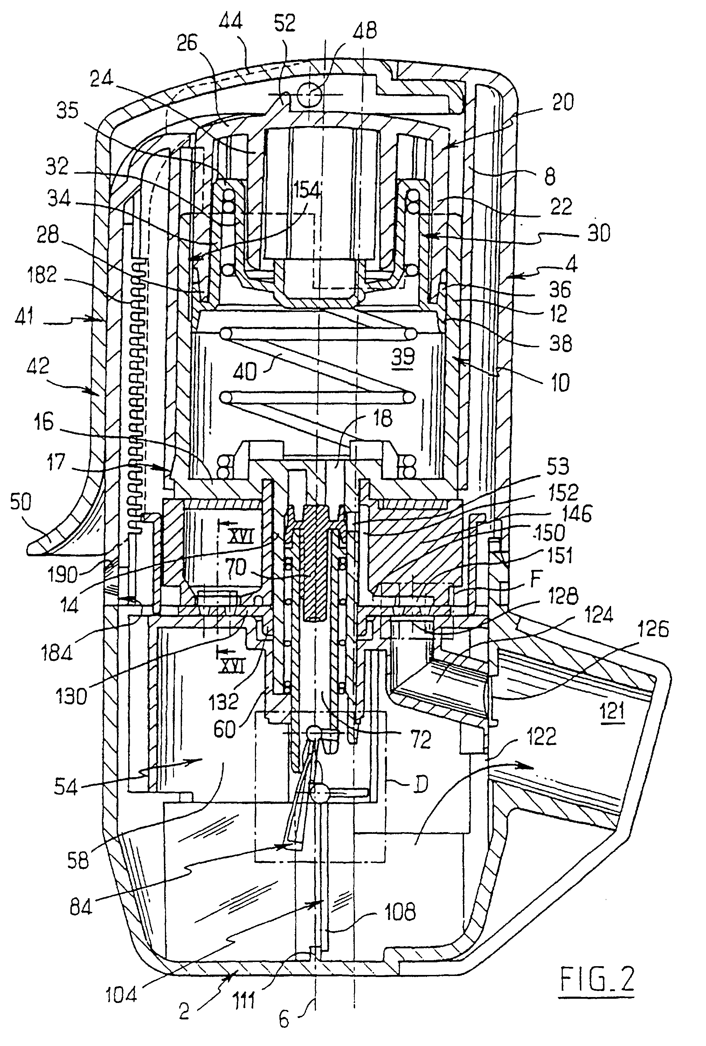

En référence aux figures 1 et 2, l'inhalateur ambulatoire pour l'administration de poudre médicinale comporte un carter comprenant un demi-carter inférieur 2 et un demi-carter supérieur 4, tous deux comportant une paroi externe de forme générale cylindrique d'axe commun 6. Les deux demi-carters sont en contact mutuel suivant un plan de joint perpendiculaire à cet axe. Le demi-carter supérieur 4 comporte une jupe interne cylindrique 8 d'axe 6 solidarisée à la paroi externe par des nervures s'étendant sur toute la hauteur de la jupe. Ces nervures assurent la rigidification du demi-carter supérieur 4.

L'inhalateur comporte également un support interne 10 comprenant un corps cylindrique 12, un moyeu cylindrique 14, tous deux creux et d'axe 6, et un fond 16 perpendiculaire à cet axe et reliant une extrémité du corps à une extrémité du moyeu. L'intérieur du moyeu communique avec l'intérieur du corps par un passage 18 ménagé dans le fond 16.

En référence à la figure 2, la face externe du corps cylindrique présente trois ergots 17 s'étendant en saillie de cette face et répartis autour de l'axe 6 au voisinage du fond 16. La jupe interne du demi-centre supérieur présente des cavités ayant une forme en creux correspondant à la forme en relief des ergots en vue de recevoir ceux-ci. Le support est fixé au demi-carter supérieur en engageant le corps 10 dans celui-ci. Les ergots et les cavités constituent des moyens de clipsage assurant la fixation du support au demi-carter, en les immobilisant relativement en translation suivant l'axe 6 et en rotation autour de cet axe.

L'inhalateur comporte un porte-piston 20 comprenant une paroi externe 22 et une jupe interne 24 cylindriques d'axe 6. Il comprend également une paroi d'extrémité supérieure 26 de forme générale plane légèrement convexe et bombée vers l'extérieur, s'étendant transversalement à l'axe 6, et à laquelle la paroi externe et la jupe sont fixées. Le porte-piston est monté à coulissement suivant l'axe 6 sur le corps 12 du support, la face interne du corps assurant le guidage de la face externe de la paroi externe du porte-piston. La paroi externe 22 du porte-piston présente un bord 28 opposé à la paroi convexe 26. Ce bord a une épaisseur (transversalement à l'axe 6) réduite par rapport à l'épaisseur du reste de la paroi externe. Le bord s'étend en continuité avec la face interne de cette paroi, et en retrait par rapport la face externe de cette paroi. Ce bord s'étend donc à distance de la face interne du corps 12.

De plus, l'inhalateur comporte un piston supérieur 30 comprenant une jupe interne 32 et une jupe externe 34, toutes deux de forme générale cylindrique d'axe 6. Le piston est enfoncé partiellement dans le porte-piston 20 entre la paroi externe 22 et la jupe 24. La jupe externe 34 du piston est en contact avec la paroi externe 22 du porte-piston, et la jupe interne 32 du piston est en contact avec la jupe 24 du porte-piston. Les deux jupes du piston sont reliées mutuellement de façon étanche à l'air par un fond annulaire 35 s'étendant à une extrémité du piston adjacente à la paroi convexe 26 du porte-piston. Les deux jupes du piston sont serrées entre la paroi et la jupe du porte-piston afin de solidariser le piston au porte-piston. La jupe externe du piston comporte à une extrémité adjacente au bord 28 un prolongement annulaire comprenant deux lèvres 36, 38 de forme tronconique orientées en sens opposés l'une de l'autre. Les deux lèvres sont en contact avec la face interne du corps 12 afin d'assurer un contact étanche à l'air du piston avec le corps. Le piston et le support délimitent ainsi une chambre de compression d'air 39. Le bord 28 du porte-piston est serré entre la lèvre 36 et la jupe externe du piston. L'inhalateur comporte également un ressort de rappel 40 d'axe 6 ayant une première extrémité en appui sur le fond 16 du support et une deuxième extrémité en appui sur le fond 35 du piston entre les deux jupes du piston. Ce ressort tend à éloigner le piston du fond 16.

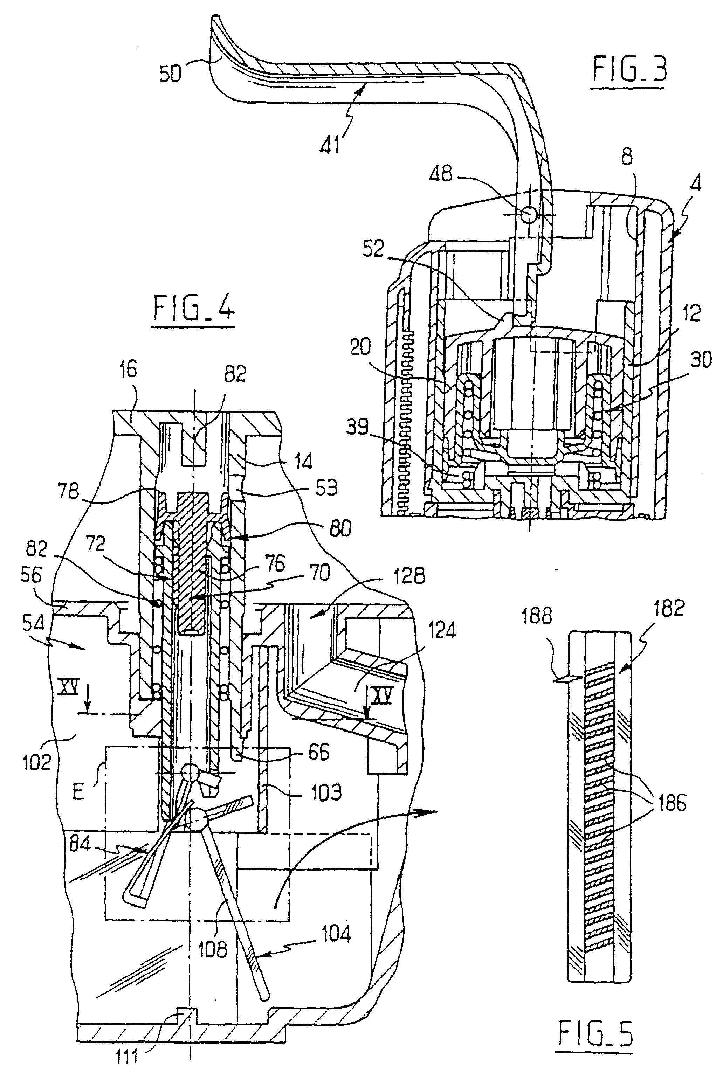

En référence aux figures 2 et 3, l'inhalateur comporte en outre un levier 41 comprenant deux tronçons 42, 44 chacun de forme générale allongée rectiligne et disposés perpendiculairement l'un à l'autre en formant un coude à environ 90°. En position de repos du levier représentée à la figure 2, le premier tronçon 42 s'étend parallèlement à l'axe 6 contre la paroi extérieure du demi-carter supérieur, et le deuxième tronçon 44 s'étend suivant une direction généralement perpendiculaire à l'axe 6 en appui sur la paroi convexe 26 du porte-piston. Le levier comporte deux tourillons 46 mutuellement coaxiaux, et la paroi extérieure du demi-carter supérieur présente des orifices dans lesquels sont engagés les tourillons, de façon à rendre le levier mobile en rotation par rapport au demi-carter supérieur suivant un axe 48 perpendiculaire à l'axe 6 s'étendant de gauche à droite et décalé vers l'arrière par rapport à l'axe 6. Les tourillons s'étendent à une portion médiane du deuxième tronçon 44. Le premier tronçon 42 présente une extrémité recourbée 50 s'étendant en direction opposée au carter en position de repos du levier. Cette extrémité facilite la préhension du levier. Lorsque l'utilisateur saisit le premier tronçon 42 et entraîne le levier en rotation dans le sens horaire sur la figure 2, une extrémité 50 du deuxième tronçon opposée au coude vient en appui sur la paroi convexe 26 du porte-piston. Cette paroi joue le rôle d'une rampe pour cette extrémité 50 lors de la rotation du levier, en transformant le mouvement de rotation du levier en un mouvement de coulissement de l'ensemble piston, porte-piston en direction du fond 16. L'actionnement du levier entraîne ainsi la compression de l'air compris dans la chambre 39. La paroi convexe 26 du porte-piston présente sur sa face externe un relief 52 disposé de sorte que l'extrémité 50 du levier vient en butée contre ce relief lorsque le deuxième tronçon 44 s'étend parallèlement à l'axe 6, définissant ainsi la fin de la course du levier. Dans cette position, le piston sollicité en direction du levier par l'air comprimé et le ressort 39, sollicite le levier suivant une direction coupant l'axe 48. Le piston ne peut donc pas actionner le levier pour le mettre en rotation. Le levier et le relief constituent ainsi des moyens de verrouillage du piston en position comprimée de la chambre, à savoir des moyens de verrouillage de la chambre dans un état comprimé. L'utilisateur n'exerce donc aucune action pour maintenir cette chambre dans un état comprimé.

En réfrence à la figure 4, le moyeu 14 présente un premier tronçon adjacent au fond 16 et de rayon interne r1, et un deuxième tronçon faisant suite au premier tronçon, distant du fond 16 et de rayon interne r2 légèrement supérieur à r1. La paroi du moyeu présente un orifice latéral 53 s'étendant perpendiculairement à l'axe 6 et situé à la jonction entre les deux tronçons.

En référence aux figures 1, 2, 14 et 15, l'inhalateur comporte une embase 54 comprenant une plaque supérieure plane 56 orientée perpendiculairement à l'axe 6 et une paroi latérale discontinue 58 constituée d'une partie arrière en forme générale de demi-cylindre d'axe 6 et d'une partie avant en secteur de cylindre d'axe 6. L'embase comprend également une cheminée centrale 60 de forme générale extérieure cylindrique d'axe 6 s'étendant à partir de la plaque 56 du même côté de celle-ci que la paroi discontinue 58. Une extrémité de la cheminée distante de la plaque 56 présente trois premières portées cylindriques internes 62 d'axe 6 et de rayon r3, que nous appellerons ici portées à moyeu, et trois deuxièmes portées cylindriques internes 64 d'axe 6 et de rayon r4 inférieur à r3, que nous appellerons ici portées à obturateur. Les portées à moyeu 62 sont décalées angulairement autour de l'axe 6 par rapport aux portées à obturateur 64 avec lesquelles elles alternent autour de l'axe 6. La jonction entre les premières et deuxièmes portées adjacentes est assurée par un épaulement discontinu 65 de l'extrémité de la cheminée. Entre la plaque 56 et cet épaulement, la cheminée présente une face interne cylindrique d'axe 6 et de rayon r3. Ce rayon r3 est égal au rayon externe du moyeu 14. L'embase 54 est montée sur le support 10, la cheminée 60 étant enfilée sur le moyeu 14. La face interne de la cheminée est en contact surface contre surface avec la face externe du moyeu. L'extrémité du moyeu 14 distante du fond 16 comporte trois ergots 66 s'étendant transversalement vers l'extérieur en saillie de la face externe du moyeu, et disposés pour s'étendre à l'extérieur de la cheminée en prenant appui axialement à l'extrémité de celle-ci. Ces ergots constituent des moyens de clipsage de l'embase 54 sur le moyeu afin de solidariser l'embase et le moyeu 14. L'embase est fixe par rapport au carter.

En référence notamment aux figures 4, 6, 7 et 8 l'inhalateur comporte un obturateur 68 comprenant un piston inférieur 70 en élastomère et un porte-piston inférieur 72. Le porte-piston inférieur 72 a une forme générale cylindrique d'axe 6. Il présente une face externe cylindrique de rayon r4 en contact surface contre surface avec les portées à obturateur 64. Le porte-piston inférieur s'étend dans le canal définit par l'intérieur du moyeu et communiquant avec la chambre. Il présente près d'une extrémité adjacente au fond 16 un bourrelet externe de rayon r2 en contact avec la face interne du moyeu. La face interne du moyeu et les portées 64 de l'embase assurent un guidage à coulissement du porte-piston inférieur 72, et donc de l'obturateur, dans le moyeu suivant l'axe 6.

Le piston inférieur 70 comporte un noyau central cylindrique 76 d'axe 6 dont la plus grande partie est emmanchée dans le porte-piston inférieur. Le noyau présente des nervures externes et le porte-piston comporte des butées internes coopérant avec les nervures pour interdire la sortie du noyau par rapport au porte-piston. Le piston est ainsi solidarisé au porte-piston inférieur. Le piston inférieur comporte un bourrelet annulaire s'étendant transversalement en saillie du noyau et en appui sur l'extrémité du porte-piston adjacente au noyau et proche du fond 16. Ce bourrelet porte deux lèvres supérieure 78 et inférieure 80 de forme tronconique et orientées en sens opposés l'une par rapport à l'autre. La lèvre supérieure 78 est adaptée à assurer le cas échéant une liaison étanche du piston 70 avec le tronçon de rayon interne r1 du moyeu, et la lèvre inférieure 80 est adaptée à assurer le cas échéant une liaison étanche du piston avec le tronçon de rayon interne r2 du moyeu. Le support 10 comporte une extension 82 s'étendant dans le passage 18 suivant l'axe 6 en constituant une butée pour l'extrémité du noyau 76 adjacente au bourrelet. Cette extension est disposée de sorte que lorsque l'obturateur se trouve en butée sur cette extension, l'orifice 53 du moyeu se trouve entre les deux lèvres 78, 80. La lèvre supérieure 78 assure alors l'étanchéité de la liaison du piston avec le moyeu. La chambre de compression d'air 39 est ainsi fermée, et la face d'extrémité du piston inférieur adjacente à la lèvre supérieure 78 est soumise à la pression régnant dans la chambre 39. Lorsque cette pression est supérieure à la pression atmosphérique, l'obturateur 68 est donc sollicité suivant l'axe 6 dans le sens de son éloignement du fond 16 du support, à savoir vers le bas sur les figures. L'inhalateur comporte un ressort de rappel 82 ayant une première extrémité en appui sur l'épaulement 65 de la cheminée et une deuxième extrémité en appui contre le bourrelet du porte-piston inférieur. Ce ressort sollicite l'obturateur suivant l'axe 6 dans le sens de son rapprochement du fond 16 du support, vers le haut sur les figures.

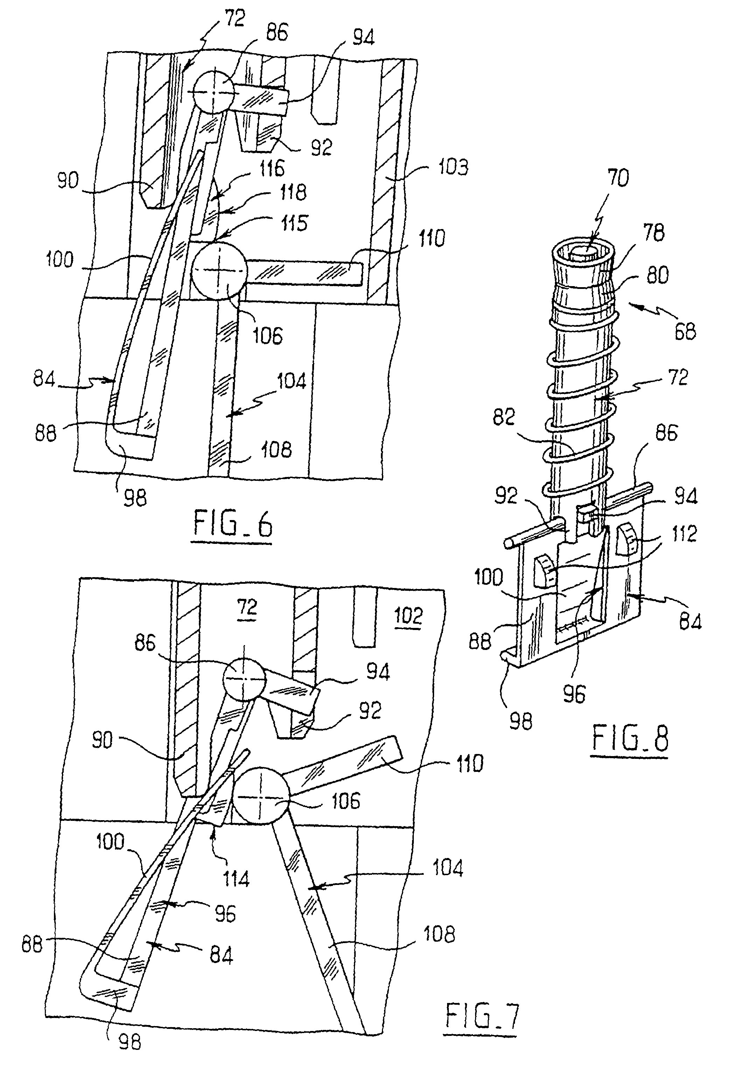

En référence aux figures 1, 2, 4, 6, 7 et 8, l'inhalateur comporte un rochet 84 comprenant un tourillon 86 et une paroi 88 de forme générale rectangulaire fixée par un bord au tourillon. Le porte-piston inférieur 72 est découpé à une extrémité opposée au piston 70 de façon à délimiter une patte arrière large 90 et deux pattes avant étroites 92 rapprochées l'une de l'autre. La découpe entre la patte arrière 90 et chacune des deux pattes avant 92 est conformée en arc de cercle, de sorte que le tourillon 86 est clipsé entre la patte arrière et les deux pattes avant. Le tourillon 86 s'étend perpendiculairement à l'axe 6 de gauche à droite en coupant cet axe. Le clipsage rend le rochet 84 mobile en rotation sur une faible amplitude par rapport au porte-piston inférieur 72 autour de l'axe du tourillon. Le rochet 84 comporte un taquet 94 fixé au tourillon et s'étendant transversalement à l'axe 6, perpendiculairement à la paroi 88 du côté des pattes avant 92 entre celles-ci. Ce taquet est agencé pour venir en butée contre la paroi du porte-piston inférieur 72 à la jonction entre les deux pattes avant 92 lorsque le rochet est disposé avec sa paroi 88 sensiblement parallèle à l'axe 6 comme représenté à la figure 6, en position de repos du rochet.

La paroi 88 du rochet présente en son centre un évidement rectangulaire 96 s'étendant sensiblement depuis le tourillon jusqu'au bord inférieur de la paroi opposé au tourillon. A ce bord, la paroi porte un bourrelet rectiligne 98 s'étendant sur toute la longueur de ce bord du côté de la paroi opposé au taquet 94. Le rochet comporte une lamelle plane flexible 100 s'étendant avant montage depuis ce bourrelet 98 parallèlement à la paroi 88. La lamelle a une forme rectangulaire voisine de celle de l'évidement 96. La lamelle s'étend en regard de cet évidement. Ses dimensions sont adaptées à permettre à une partie supérieure de la lamelle de traverser l'évidement sous l'effet d'une sollicitation. La lamelle 100 et la patte arrière 90 du porte-piston inférieur sont disposées de sorte que la patte arrière sollicite en permanence la lamelle et provoque sa flexion. La lamelle traverse donc toujours partiellement l'évidement. Dans la position de repos du rochet représentée à la figure 6 avec la paroi 88 sensiblement parallèle à l'axe 6, la patte arrière sollicite la lamelle au minimum. La sollicitation est plus intense, et donc la flexion de la lamelle plus prononcée, lorsque le rochet est basculé vers l'arrière, à savoir dans le sens horaire comme sur la figure 7.

L'embase 54 comporte deux parois planes 102 parallèles entre elles s'étendant à partir de la plaque 56 en regard de la cheminée parallèlement à l'axe 6 en étant orientées d'avant en arrière. L'embase comporte également un panneau plan rectangulaire 103 s'étendant d'une paroi 102 à l'autre perpendiculairement à celles-ci et parallèlement à l'axe 6, en avant de la cheminée 60, en étant orienté de gauche à droite.

L'inhalateur comporte un volet 104 comprenant un tourillon 106 et un panneau 108 de forme générale rectangulaire fixé par un bord au tourillon. Les extrémités du tourillon sont clipsées dans des découpes en arc de cercle du bord inférieur des deux parois 102. Le tourillon 106 s'étend parallèlement au tourillon 86 du rochet, dans le prolongement du porte-piston inférieur 72 en étant légèrement décalé vers l'avant par rapport au tourillon 86 du rochet, à savoir en direction des pattes avant 92. Le tourillon 106 s'étend dans le prolongement de l'obturateur. Le volet 104 est mobile en rotation par rapport à l'embase autour de l'axe du tourillon 106. Le panneau 108 est fixé au tourillon 106 en étant décalé latéralement vers l'avant par rapport à l'axe du tourillon. Le volet comporte une casquette plane rectangulaire 110 fixée par un bord au tourillon et disposée perpendiculairement au panneau 108 vers l'avant. La casquette est agencée de sorte que lorsque le volet est au repos avec le panneau 108 sensiblement parallèle à l'axe 6, la casquette s'étend entre les parois 102 et le panneau 103 de l'embase en obturant grossièrement l'espace délimité par ceux-ci du côté opposé au panneau 108 du volet. Une obturation totale n'est pas nécessaire. Le demi-carter inférieur comporte une butée 111 pour le panneau 108 limitant la rotation vers l'arrière du volet et définissant la position de repos du volet avec la paroi 108 verticale.

La paroi 88 du rochet porte sur sa face avant deux ergots 112 disposés de part et d'autre de l'évidement 96 à proximité du tourillon 86. Chaque ergot 112 présente une face inférieure plane 114 perpendiculaire à la paroi 88 et parallèle au tourillon 86, deux faces latérales planes 116 parallèles entre elles et perpendiculaires à la paroi 88 et au tourillon, et une face courbe convexe 118 s'étendant à partir de la paroi 88 jusqu'à la face inférieure 114 en formant une arête vive 115 à environ 90° avec celle-ci. Les ergots sont disposés de sorte que lorsque l'obturateur 68 est en butée sur l'extension 82, les ergots reposent par leur arête vive sur le tourillon 106 du volet comme le montre la figure 6. L'arête vive des ergots se trouve à proximité du sommet du tourillon en étant légèrement décalée vers l'arrière en direction de la paroi 88 du rochet. Cette position est définie par le taquet 94 en butée contre le porte-piston inférieur et maintenue par la coopération de la patte arrière 90 et de la lamelle 100 tant que le volet reste en position de repos.

Le demi-carter inférieur comporte deux déflecteurs plans 120 parallèles entre eux s'étendant verticalement parallèlement à l'axe 6 à partir de la face inférieure du demi-carter en direction de l'embase 54. Les déflecteurs 120 s'étendent dans le prolongement des deux parois 102 de l'embase, en continuité respectivement avec celles-ci comme le montre la figure 1. Un bord supérieur de chaque déflecteur est en contact avec le bord inférieur de la paroi 102 associée de façon sensiblement étanche à l'air. La distance entre les deux déflecteurs 120 est très légèrement supérieure à la longueur du panneau 108 du volet parallèlement au tourillon 106, de sorte que le volet 104 est libre de tourner autour de son axe de rotation, une certaine étanchéité à l'air étant assurée entre le volet et les déflecteurs.

En référence aux figures 2 et 4, le demi-carter inférieur 2 comporte sur sa face avant une embouchure d'inhalation 121 pour un patient, mettant l'intérieur de l'inhalateur en communication avec l'extérieur. L'embouchure s'étend suivant une direction généralement perpendiculaire à l'axe 6 en étant légèrement orientée vers le bas. La partie avant de la paroi latérale 58 de l'embase s'étend en regard de cette embouchure. Sa dimension suivant l'axe 6 est suffisamment réduite pour ménager un espace 122 de communication entre l'embouchure 121 d'une part, et l'embase 54 et le demi-carter inférieur 2 d'autre part jusqu'au volet du côté de la face avant du panneau 108. La partie arrière de la paroi latérale 58 de l'embase s'étend à distance de la paroi latérale arrière du demi-carter inférieur. L'espace ainsi ménagé est en communication avec l'espace délimité entre la paroi du demi-carter supérieur 4 et la jupe 8, cet espace communiquant avec l'extérieur par le sommet du demi-carter supérieur. La face arrière du panneau 108 du volet est donc également en communication avec l'extérieur.

L'embase comporte un canal 124 comprenant deux tronçons s'étendant dans le prolongement l'un de l'autre et formant un coude. Le premier tronçon s'étend immédiatement en amont de l'embouchure parallèlement à celle-ci de façon à déboucher dans l'embouchure. L'extrémité libre de ce tronçon porte une grille 126 de fractionnement de la poudre à administrer au patient. La grille s'étend transversalement à l'axe du tronçon. Le deuxième tronçon est fixé à la plaque supérieure 56 de l'embase, en regard d'un orifice circulaire 128 de celle-ci dans lequel il débouche. Le deuxième tronçon est sensiblement parallèle à l'axe 6, à savoir vertical ici.

En référence aux figures 1, 2, 11 et 12, l'inhalateur comporte un plateau 129 assurant le transfert et le dosage de la poudre. Ce plateau comprend un disque circulaire 130 d'axe 6 présentant un orifice central, le disque étant prolongé axialement vers le bas au niveau de l'orifice par une bague 132. Par cet orifice central, le plateau est enfilé sur le moyeu 14 en contact surface contre surface avec celui-ci. La face inférieure du disque s'étend sur la plaque 56 de l'embase. Dans le disque sont ménagées des alvéoles 134 traversant l'épaisseur du disque suivant l'axe 6. Ces alvéoles sont réparties en dix groupes de six alvéoles. Les alvéoles de chaque groupe sont disposées en cercle. Le volume de chaque groupe d'alvéoles correspond à une dose de poudre à administrer. L'orifice 128 de l'embase a un diamètre suffisant pour envelopper les diamètres des alvéoles d'un même groupe. Les groupes sont disposés en un cercle centré sur l'axe 6. Les alvéoles ont un profil parallèlement à l'axe 6 de forme tronconique, la section étroite du cône se trouvant du côté de la plaque 56 de l'embase, à savoir du côté aval des alvéoles par rapport au sens de l'écoulement de l'air comprimé comme on le verra. Les alvéoles ont ainsi une section transversale diminuant vers l'aval.

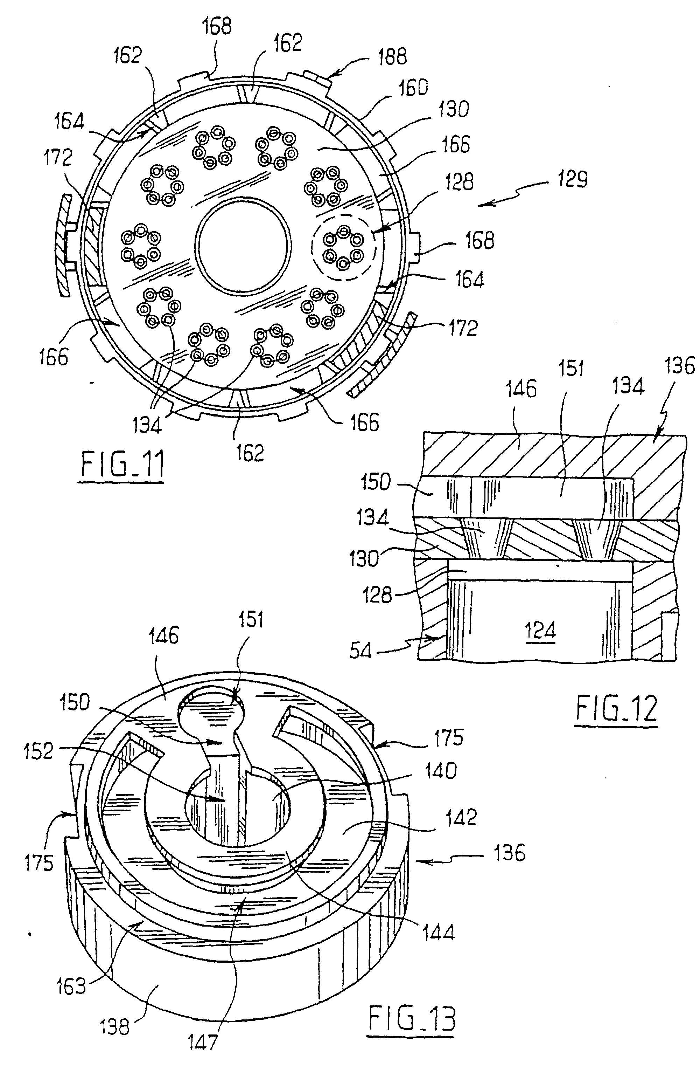

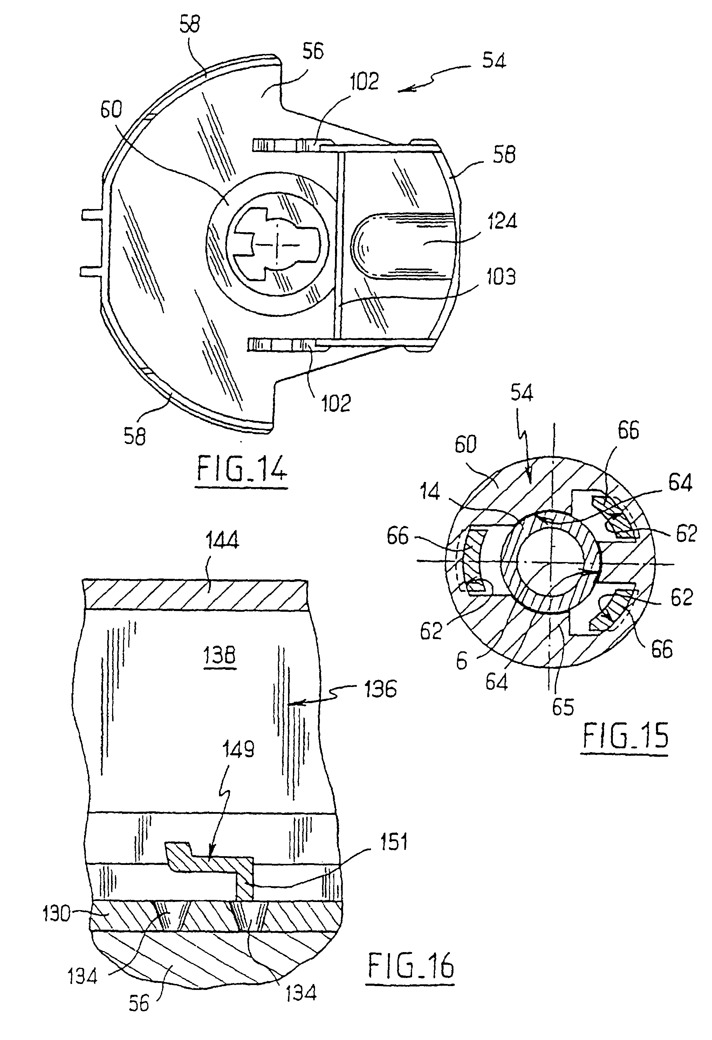

En référence aux figures 1, 2 et 13, l'inhalateur comporte aussi une réserve 136 de poudre destinée à être administrée au patient. Cette réserve comprend une paroi cylindrique périphérique 138 et une paroi cylindrique centrale 140, toutes deux d'axe 6. Elle comprend deux parois circulaires supérieure 142 et inférieure 144. Pour la clarté de la représentation, la réserve a été retournée sur la figure 13 de sorte que la .position des parois circulaires est inversée. La paroi cylindrique centrale 140 est en contact avec le moyeu 14 sur lequel la réserve est enfilée avec un ajustement légèrement serré entre la paroi et le moyeu pour éviter toute intrusion de poudre entre la paroi et le moyeu et toute perte d'air lors de la libération de l'air comprimé comme on le verra. La réserve est immobilisée par rapport au moyeu. La face supérieure de la paroi supérieure 142 est en contact avec le fond 16 du support. La face inférieure de la paroi inférieure 144 est en contact avec le disque 130 du plateau. La réserve est généralement creuse entre ses quatre parois, sauf sur un secteur angulaire 146 d'écartement supérieur à un groupe d'alvéoles, où la réserve est réalisée en étant pleine comme le montrent les figures 2 et 13. La paroi circulaire inférieure 144 présente un évidement 147 en arc de cercle interrompu au niveau du secteur plein 146. La réserve a ainsi une forme générale torique et définit une enceinte remplie de poudre. La paroi circulaire supérieure 142 est constituée par un couvercle amovible fixé à la réserve de façon étanche à la poudre et permettant de remplir la réserve. La poudre de la réserve traverse l'évidement 147 par gravité et se dépose dans les alvéoles 134 du plateau. Le remplissage des alvéoles avec la poudre s'effectue dans le sens de la traversée des alvéoles par l'air comprimé, de l'amont vers l'aval. Comme on le verra, le plateau est mobile en rotation autour de l'axe 6 par rapport à la réserve. La réserve comporte des racleurs 149 visibles notamment sur la figure 16 et non représentés sur la figure 13. Ces racleurs comportent une lame 151 généralement radiale et parallèle à l'axe 6, traversant l'évidement 147, en appui élastique contre la face supérieure du disque 130 du plateau et s'étendant dans la trajectoire des groupes d'alvéoles. Au passage d'un racleur, la poudre est tassée dans les alvéoles. La plaque supérieure 56 de l'embase s'étend contre la face inférieure du disque 130 et interdit la sortie de la poudre des alvéoles, sauf au niveau de l'orifice 128 lors de l'administration de la poudre comme on le verra. Le disque s'étend ainsi entre la face inférieure 144 de la réserve et la plaque 56 de l'embase.

Au niveau du secteur plein 146 de la réserve, la paroi circulaire inférieure 144 présente un renfoncement circulaire 151 de même forme et de mêmes dimensions que l'orifice 128 de l'embase. Ce renfoncement demeure en regard de l'orifice de l'embase. Il est prolongé en direction de l'axe 6 par une courte gorge 150. La paroi cylindrique centrale 140 présente une deuxième gorge 152 à profil rectangulaire suivant la direction transversale à l'axe 6. Cette gorge 152 s'étend parallèlement à l'axe 6, depuis la gorge 150 avec laquelle elle communique jusqu'en regard de l'orifice 53 du moyeu, en s'interrompant avant la paroi circulaire supérieure 142 de la réserve.

On va maintenant expliquer le fonctionnement de l'inhalateur en ce qui concerne les éléments qui ont été décrits jusqu'ici.

Au repos, l'inhalateur se trouve dans la position représentée aux figures 1, 2 et 6. Le levier 41 est en appui contre le demi-carter supérieur 4. Le piston supérieur 30 est sollicité vers le haut par le ressort 40 et se trouve en position haute en butée contre le levier. L'intérieur de la chambre de compression 39 se trouve à la pression atmosphérique. L'obturateur 68 est sollicité vers la haut par le ressort 40 et se trouve en position haute, en butée contre l'extension 82. La lèvre supérieure 78 du piston inférieur est appliquée de façon étanche à l'air contre le tronçon supérieur de petit rayon interne r1 du moyeu. L'obturateur obture ainsi l'orifice 53 de la chambre de compression 39. La lamelle 100 du rochet est sollicitée vers l'avant par la patte arrière 90. Le taquet 94 est en butée contre le porte-piston inférieur. L'arête vive 115 des ergots est en contact avec le tourillon 106 du volet. Le volet s'étend avec son panneau 108 parallèle à l'axe 6 sous l'effet de la gravité.

Pour l'administration d'une dose de poudre, le patient fait pivoter le levier dans le sens horaire jusqu'à la position représentée à la figure 3. Ce pivotement entraîne le coulissement du porte-piston supérieur 26 et du piston supérieur 30 jusqu'à sa position basse proche du fond 16. Ce coulissement génère une augmentation de la pression de l'air dans la chambre 39. En effet, le rochet 84 en appui sur le volet 104 ne peut pas coulisser vers le bas. L'obturateur 68 est donc lui aussi immobilisé. Lorsque le piston supérieur 30 arrive en fin de course en position basse, l'air de la chambre 39 est comprimé à la pression maximale. Cette pression sollicite la face supérieure de l'obturateur vers le bas en direction du volet. Cette sollicitation se traduit par le fait que les ergots 112 appuient sur le volet avec leur arête vive 115 tout en demeurant immobilisés, ce qui interdit le coulissement de l'obturateur. Le levier en position relevée verrouille le piston supérieur 30 en position basse. L'inhalateur est ainsi mis en charge et le demeure sans action du patient jusqu'à ce que le patient déclenche l'administration du traitement. Comme on le verra dans la suite, un groupe d'alvéoles se trouve à ce moment en regard de l'orifice 128 de l'embase. Le tassement de la poudre dans les alvéoles et la forme des alvéoles empêchent la poudre de sortir naturellement des alvéoles en traversant le plateau, par exemple par gravité.On remarque que le mécanisme à volet est mis en charge par la pression de l'air comprimé dans la chambre, sans autre moyen tel qu'un ressort de mise en charge.

Le patient applique sa bouche autour de l'embouchure 121 de façon à pouvoir aspirer dans l'embouchure. Le patient aspire. Cette aspiration provoque une dépression du côté de la face avant du volet 104. Le volet est mis en rotation vers l'avant, dans le sens anti-horaire sur les figures 4 et 7. L'amorce de la rotation du tourillon 106 du volet provoque l'échappement de l'arète vive 115 des ergots et la suppression de l'appui. Sous la sollicitation de l'obturateur soumis à la pression de la chambre, le rochet est entraîné en rotation autour de son tourillon 86 vers l'arrière dans le sens horaire, tandis que l'obturateur coulisse suivant l'axe 6 en direction du volet. Le rochet sollicité via la lamelle 100 par la patte arrière 90 du moyeu est placé en appui contre le tourillon 106 du volet par les faces courbes 118 des ergots. Dès le début du coulissement de l'obturateur, la lèvre inférieure 80 du piston inférieur vient en contact étanche à l'air avec le deuxième tronçon de grand rayon interne r2 du moyeu. De plus, le contact étanche de la lèvre supérieure 78 du piston inférieur avec le moyeu est interrompu. L'obturateur se trouve éloigné de l'orifice. L'air comprimé de la chambre 39 s'échappe par l'orifice 53 du moyeu, suit la gorge 152, puis la gorge 150 de la réserve à l'extérieur du moyeu, puis aboutit dans le renfoncement 151. Ici, il force la poudre du groupe d'alvéoles disposé au niveau du renfoncement à traverser le disque 130 et entraîne cette poudre dans le canal 124 de l'embase. La poudre est fragmentée par le flux d'air, puis au passage de la grille 126 s'étendant en aval du plateau. L'air transportant la poudre traverse l'embouchure 121 et aboutit dans les poumons de l'utilisateur. La libération de l'air comprimé est ainsi déclenchée par l'aspiration du patient. Le flux d'air comprimé libéré traverse les alvéoles verticalement de bas en haut. Le flux d'air comprimé s'ajoute donc au poids de la poudre pour transporter celle-ci et la fragmenter.

Dès que l'aspiration du patient cesse, le volet 104 pivote vers l'arrière par gravité pour revenir à sa position de repos. Le ressort 82 de l'obturateur est agencé de sorte qu'il rappelle l'obturateur vers le haut en direction du fond 16 dès qu'une pression voisine de la pression atmosphérique règne dans la chambre de compression après échappement de l'air comprimé. Le coulissement vers le haut de l'obturateur jusqu'à sa mise en butée contre l'extension 82 entraîne l'obturation de l'orifice 53. A la fin de ce coulissement, la patte arrière 90 du moyeu sollicitant la lamelle 100, pousse le rochet pour mettre les ergots 112 en contact par leur arête vive avec le tourillon du volet comme précédemment.

Lorsque le patient abaisse à nouveau le levier dans la position de repos, le ressort de rappel 40 entraîne le retour du piston supérieur 30 vers sa position haute de repos. L'inhalateur est alors prêt pour une nouvelle utilisation. Le ressort 40 a une raideur suffisamment faible pour permettre au patient de mettre l'inhalateur en charge sans effort. Dès lors, il se peut que ce ressort développe une force de réaction d'intensité insuffisante pour pousser le piston sur le dernier 1/6 de sa course jusqu'en position haute, du fait de la dépression se produisant alors dans la chambre 39 à nouveau obturée. C'est pourquoi il est avantageux de réaliser dans la face interne du corps 12 du support une rainure 154 s'étendant parallèlement à l'axe 6 depuis l'extrémité supérieure libre du corps sur une longueur suffisant à interrompre le contact étanche des lèvres 36, 38 du piston supérieur avec cette face interne après que le piston a parcouru 5/6 de sa course. Sur le dernier 1/6 de course, l'air extérieur s'introduit par la rainure 154 dans la chambre 39, ce qui supprime la dépression. Le ressort peut alors pousser le piston jusqu'à sa position haute.

On voit que l'inhalateur comporte un premier conduit définit par les gorges 152 et 150, le canal 124 et l'embouchure 121, et traversant l'orifice 128. Ce conduit est destiné à être parcouru par l'air comprimé s'échappant de la chambre la dose de poudre à administrer étant dispersée dans ce conduit. On voit aussi que l'inhalateur comporte un deuxième conduit délimité entre le demi-carter supérieur 4 et la jupe 8, le demi-carter inférieur 2 et l'embase 54 et l'embouchure 121, et traversant l'espace 122. Ce conduit est distinct du premier. Le volet et le rochet s'étendent dans ce conduit. Le volet s'étend hors de la trajectoire de la poudre et ne risque pas de gêner son acheminement par le flux d'air. Le mécanisme constitué par le rochet et le volet ne risque pas d'être altéré par des dépôts de poudre.

L'ajustement précité entre le volet et les parois 120 entraîne qu'une faible aspiration de la part du patient provoque le déplacement du volet. La sensibilité du déclenchement du volet est fonction de la qualité de l'ajustement.

Le volet et le rochet constituent des moyens pour commander l'éloignement de l'obturateur 68 par rapport à l'orifice 53 en vue de mettre fin à son obturation et de le laisser libre pour l'échappement de l'air comprimé de la chambre 39. Ces moyens sont agencés pour être actionnés par une aspiration créée dans l'embouchure 121. Le mécanisme à volet, comportant trois pièces mobiles les unes par rapport aux autres (le volet, le rochet et l'obturateur) est de structure relativement simple.

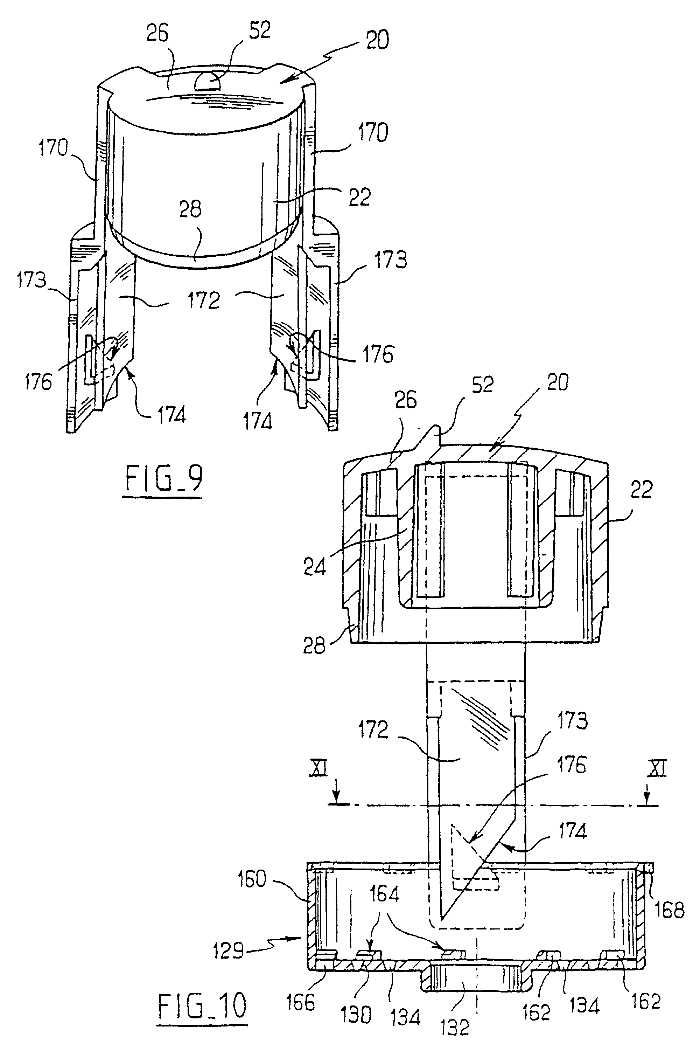

En référence aux figures 1, 10 et 11, le plateau comporte une paroi latérale cylindrique 160 d'axe 6 s'étendant sur le pourtour du disque 130, d'un côté du disque vers le haut en regard de la paroi périphérique 138 de la réserve. Le disque porte dix bossages 162 régulièrement répartis sur le pourtour du disque en étant contigus intérieurement à la paroi 160. Ces bossages s'étendent en saillie de la face supérieure du disque, en regard de la réserve. Celle-ci présente un épaulement 163 à la jonction entre la paroi périphérique 138 et la paroi inférieure 144. Cet épaulement constitue un logement pour les bossages. Les bossages sont profilés suivant la direction radiale à l'axe 6. Ils présentent un profil rectangulaire, une arête supérieure du rectangle étant chanfreinée pour définir une facette 164 inclinée vers le haut et en direction du bossage suivant dans la succession. Le disque présente des évidements 166 en arc de cercle ménagés entre les bossages successifs. La paroi latérale 160 du plateau porte à son bord supérieur dix tétons 168 de forme rectangulaire, s'étendant radialement en saillie de la face externe de la paroi. Les tétons se trouvent au niveau des évidements 166 respectifs, en vue en plan.

En référence aux figures 1, 9 et 10, le porte-piston supérieur comporte deux extensions latérales 170 s'étendant depuis la paroi supérieure 26 en regard de la paroi latérale 22 parallèlement à l'axe 6. Les deux extensions forment avec l'axe 6 un angle d'environ 150 degrés. A une extrémité s'étendant au-delà du bord inférieur 28 de la paroi latérale 22, chaque extension se dédouble en deux bras allongés 172, 173 de forme cylindrique d'axe 6 concentriques entre eux et disposés en regard l'un de l'autre, s'étendant suivant l'axe 6.

Le premier bras 172 s'étend dans le prolongement de l'extension 170. Il présente une extrémité inférieure biseautée définissant une facette 174 inclinée vers le bas et latéralement. Les deux facettes 174 des bras 172 sont orientées en sens contraires. Les bras 172 s'étendent en regard de la face interne de la paroi latérale 160 du plateau. La largeur d'une partie supérieure du premier bras est identique à celle de l'extension associée, et la largeur d'une partie inférieure du premier bras est très légèrement inférieure à la longueur des évidements 166 du disque. Le bras est dimensionné pour pouvoir s'engager dans ces évidements, comme le montre la figure 11. La paroi périphérique 138 de la réserve 136 présente deux encoches 175 s'étendant suivant l'axe 6, ayant même largeur et même épaisseur que les premiers bras 172, et disposées en formant un angle identique avec l'axe 6. Les bras 172 sont logés dans ces encoches qui permettent le coulissement des bras par rapport à la réserve parallèlement à l'axe 6.

Le deuxième bras 173 de chaque extension est décalé vers l'extérieur par rapport à l'axe 6 et s'étend en regard de la face externe de la paroi latérale 160 du plateau. Il porte sur sa face interne un relief présentant en élévation une forme générale de triangle rectangle. L'hypoténuse du triangle constitue une facette 176 inclinée vers la haut et latéralement, du même côté que la facette 174 du premier bras associé. Les facettes 176 des deuxièmes bras 173 sont orientées en sens contraires. Le relief se trouve en regard de la pointe biseautée du premier bras 172 associé. La largeur du relief est inférieure à la distance séparant deux tétons 168 du plateau, de sorte que le relief peut traverser l'espace séparant deux tétons 168 consécutifs.

En référence à la figure 1, le corps 10 du support présente deux gorges 180 s'étendant sur la face externe de la paroi latérale 12 parallèlement à l'axe 6 depuis l'extrémité supérieure ouverte du corps jusqu'au fond 16. Les gorges 180 forment avec l'axe 6 un angle identique à celui des extensions 170. La largeur de chaque gorge 180 est égale à la largeur de l'extension 170 et de 1 partie supérieure du premier bras 172 qui la prolonge, de sorte que chaque extension 170 et son bras 172 sont logés partiellement suivant leur épaisseur dans la gorge 180 correspondante. Les gorges 180 participent ainsi au guidage à coulissement du porte-piston supérieur 20 par rapport au support 10 parallèlement à l'axe 6. De plus, ces gorges interdisent la rotation du porte-piston supérieur par rapport au support autour de l'axe 6. Le coulissement des premiers bras 172 dans les encoches 175 interdit la rotation de la réserve 136 par rapport au porte-piston supérieur 20 autour de l'axe 6, et par voie de conséquence par rapport au support 10.

L'extrémité des bras 172 est disposée de sorte que en position de repos de l'inhalateur, à savoir lorsque le piston est en position haute, la facette 174 s'étend au-dessus des bossages 162, la portion basse de la facette 174 se trouvant à la verticale de la facette 164 de l'un des bossages, la facette du bossage se trouvant dans la trajectoire de la facette 174. Ainsi, le coulissement du porte-piston supérieur vers le bas sous l'action du levier provoque la rencontre de la facette 174 du bras avec la facette 164 du bossage, orientées alors parallèlement l'une à l'autre. La facette 174 faisant office de rampe pour la facette 164 du bossage, la poursuite du coulissement du porte-piston supérieur entraîne la rotation du plateau autour de l'axe 6. Lorsque la facette 164 du bossage quitte cette rampe, la rotation du plateau cesse, et le bras 172a pénètre dans l'évidement 166 adjacent à la facette 164 du bossage. Le plateau se trouve alors immobilisé en rotation par rapport au porte-piston supérieur autour de l'axe 6. Le porte-piston supérieur et le plateau sont agencés de sorte que ce mouvement du plateau par effet de rampe place un groupe d'alvéoles en regard de l'orifice 128 de l'embase.

La fin de la course vers le bas du porte-piston supérieur correspond à l'état comprimé de la chambre 39. Le relief du bras 173 est disposé de sorte que la facette 176 de ce bras s'étend alors au-dessous des tétons 168. La portion haute de la facette 176 se trouve à la verticale d'un bord de l'un des tétons 168, le téton étant dans la trajectoire de la facette. Lorsque le patient abaisse le levier en position de repos, le porte-piston supérieur coulisse vers le haut, ce qui provoque la rencontre de la facette 176 du relief avec le bord du téton 168 en regard. La facette 176 faisant office de rampe pour le bord du téton, la poursuite du coulissement vers le haut du porte-piston supérieur entraîne la rotation du plateau autour de l'axe 6, dans le même sens que précédemment. Pendant ce mouvement de rotation du plateau, le bras 172 sort progressivement de l'évidement 166, la rotation étant permise par la forme en pointe de l'extrémité du bras 172. Le porte-piston supérieur et le plateau sont agencés de sorte que ce nouveau mouvement de rotation du plateau éloigne de l'orifice 128 le groupe d'alvéoles précité, maintenant exemptes de poudre. La rotation du plateau s'interrompt lorsque le téton quitte la facette 176. Le groupe d'alvéoles s'étend alors entre la réserve et la plaque 56 de l'embase. Le porte-piston supérieur et le plateau sont agencés de sorte que lorsque le porte-piston supérieur est revenu dans la position de repos de la figure 1, la facette 174 se trouve à la verticale du prochain bossage 162.

Le coulissement vers le bas du porte-piston supérieur dispose donc un groupe d'alvéoles en regard de l'orifice 128. Ensuite, le coulissement vers le haut du porte-piston supérieur dispose ce groupe d'alvéoles à l'abris de l'orifice 128 et de l'air ambiant. Ces deux mouvements du plateau sont commandés par l'actionnement du levier. Ainsi, l'actionnement du levier à partir de la position de repos provoque simultanément la mise en compression de la chambre et le positionnement d'un groupe d'alvéoles remplies de poudre en regard de l'orifice 128. Le retour du levier en position de repos entraîne la mise à l'abri de ce groupe vidé de sa poudre. Un avantage de cet inhalateur est que les alvéoles, vides ou pleines, ne sont au contact de l'air ambiant qu'au moment de l'utilisation de l'inhalateur, à savoir lorsque le levier est levé. Leur propreté à l'égard d'éléments étrangers est donc préservée.

En référence aux figures 2 et 5, l'inhalateur comporte une réglette rectiligne 182 s'étendant parallèlement à l'axe 6. La paroi latérale arrière des demi-carters supérieur et inférieur présente sur sa face interne une gorge 184 adaptée à recevoir la réglette à coulissement suivant l'axe 6. La réglette présente sur sa face dirigée vers l'axe 6 et opposée à la gorge une denture en crémaillère constituée de dents rectilignes 186 alignées et parallèles entre elles. Les dents sont légèrement inclinées par rapport à la direction transversale à l'axe 6. Le sommet des dents a une forme cylindrique d'axe 6. L'un des tétons 168 du plateau porte une dent de comptage 188 s'étendant en saillie d'une facette du téton opposée à la paroi 160. Cette dent a une forme de losange adaptée à s'insérer entre deux dents 186 successives de la règlette lors de la rotation du plateau. Le passage de la dent de comptage entraîne le coulissement de la réglette vers le bas et positionne la réglette de sorte qu'au prochain passage, la dent de comptage s'insérera dans l'espace interdentaire suivant. La face arrière de la réglette en contact avec la gorge présente une marque (non représentée) près de son extrémité supérieure. Le demi-carter supérieur présente une fenêtre 190 ménagée dans sa paroi arrière au fond de la gorge 184 dans la trajectoire de la marque. La marque et la fenêtre sont disposées de sorte que la marque est visible de l'extérieur à travers la fenêtre lorsque la dent de comptage s'est engagée dans l'espace entre les deux dents les plus hautes, ce qui correspond à la fin de la course vers le bas de la réglette.

Chaque utilisation de l'inhalateur entraîne la rotation du plateau sur 1/10 de tour. A la fin de chaque tour, la dent de comptage provoque le coulissement vers le bas de la réglette. Chaque dent 186 de la règlette correspond ainsi à dix utilisations. Le nombre de dents est choisi de sorte que la marque apparaît dans la fenêtre lorsque la réserve est vide. La réglette et le plateau constituent un système de comptage des utilisations, permettant de compter avec précision un grand nombre d'utilisations et présentant un encombrement réduit.

Sont concentriques d'axe 6 les demi-carters inférieur et supérieur, le piston supérieur, le porte-piston supérieur, le ressort supérieur, le support, le piston inférieur, le porte-piston inférieur, le ressort inférieur, la réserve, le plateau, le rochet et l'embase. Cette caractéristique diminue l'encombrement total des pièces et réduit le volume de l'inhalateur. L'inhalateur étant ambulatoire, cela est particulièrement avantageux.

Le montage de l'inhalateur est effectué de la façon suivante. On assemble le piston inférieur et le porte-piston inférieur. On enfile le ressort inférieur sur ce dernier et on clipse le rochet sur le porte-piston inférieur. On enfile sur le moyeu 14 la réserve et le plateau, et on enfile dans le moyeu l'obturateur. On clipse le volet sur l'embase 54. On enfile l'embase sur le moyeu en clipsant l'embase sur le moyeu, ce qui solidarise les pièces précitées. On dispose le ressort supérieur dans le corps 10. On fixe le piston supérieur au porte-piston supérieur par serrage. On engage les extensions du porte-piston supérieur dans les gorges 180 et on engage le porte-piston supérieur sur le support. On dispose la réglette dans la gorge 184 du demi-carter supérieur. On fixe le levier au demi-carter supérieur On dispose l'ensemble comprenant le support dans l'un des demi-carters, et on clipse les deux demi-carters entre eux, ceux-ci étant munis à cette fin de moyens de clipsage adaptés.

Le montage de l'inhalateur est simple et rapide, notamment en raison de la présence du support 10 commun à de nombreuses pièces. Ce support permet d'assembler entre elles les pièces concernées avant leur introduction dans le carter. Le clipsage des pièces facilite également ce montage.

Toutes les pièces à l'exception des ressorts, de la grille, et du piston inférieur peuvent être réalisées en matière plastique moulée, ce qui donne à l'inhalateur un coût réduit.

Bien entendu, on pourra apporter à l'invention de nombreuses modifications sans sortir du cadre de celle-ci.

En particulier, le mécanisme de commande de l'obturateur 68 pourra être de structure différente. Il pourra comprendre un capteur électronique de dépression dans l'embouchure. Le mécanisme à volet pourra être d'un type différent. Le mécanisme à volet pourra faire partie d'un inhalateur d'un type différent, par exemple un inhalateur à bande de doses de poudre, un inhalateur avec réserve permanente de gaz sous pression, ou un inhalateur à aérosol.

Les alvéoles pourront avoir une section transversale diminuant vers l'aval, sans que leur forme soit nécessairement tronconique. Les caractéristiques concernant la forme des alvéoles pourront être appliquées à des inhalateurs à poudre de structures différentes.

Le positionnement des alvéoles en regard de l'orifice pourra être commandé par des moyens séparés, indépendamment de la mise en compression de la chambre par le levier.

Le principe de montage de certaines des pièces sur un support 10 commun avant introduction dans le carter pourra être appliqué pour des inhalateurs de structures différentes, par exemple à aérosol. Les pièces pourront être fixées entre elles autrement que par clipsage.

Le moyen de commande de la mise en compression de la chambre et/ou du positionnement des alvéoles pourra être différent d'un levier. Il pourra s'agir d'un bouton molleté.

On pourra modifier le nombre des groupes d'alvéoles, et le nombre d'alvéoles de chaque groupe, ainsi que la disposition des alvéoles de chaque groupe. Pour améliorer la fragmentation de la poudre, il sera avantageux de réduire la surface de la paroi du plateau s'étendant entre les alvéoles en regard de l'orifice 128 lors de l'utilisation, ou bien en augmentant le nombre d'alvéoles et en réduisant leur taille, ou bien en réduisant le diamètre de l'orifice 128 et en rapprochant les alvéoles entre elles. On pourra remplacer le disque du plateau par une grille, par exemple à structure en nid-d'abeilles dont les alvéoles constitueront les alvéoles de dosage de la poudre.

Dans la description qui précède, l'inhalateur comporte des moyens de rappel du rochet vers une position d'appui sur le volet.

## Revendications

1. Inhalateur à poudre comportant une chambre d'alimentation en air (39), un conduit communiquant avec la chambre par un orifice (53) et débouchant dans une embouchure d'inhalation (121), des moyens (136, 130) pour disposer une dose de poudre dans le conduit, et un détecteur (104) d'une aspiration créée dans l'embouchure, la chambre constituant une chambre de compression d'air, et ledit inhalateur comportant en outre :
- un obturateur mobile (68) agencé pour pouvoir obturer l'orifice ;
- des moyens (84, 104) pour éloigner l'obturateur (68) par rapport à l'orifice, commandés par le détecteur (104) ;
- des moyens (30), à gaz comprimé, de sollicitation de l'obturateur (68) dans le sens de son éloignement de l'orifice lorsqu'il obture l'orifice ;
- un mécanisme (84) d'immobilisation de l'obturateur (68) à l'encontre des moyens de sollicitation (30) dans une position d'obturation de l'orifice, ledit inhalateur étant
**caractérisé en ce que** les moyens de sollicitation (30) sont aptes à solliciter l'obturateur (68) en position d'obturation avant la détection de l'aspiration par le détecteur (104).

2. Inhalateur selon la revendication 1, **caractérisé en ce que** le mécanisme d'immobilisation (84, 104) fait partie des moyens pour éloigner l'obturateur.

3. Inhalateur selon la revendication 1 ou 2, **caractérisé en ce qu'**il comporte un deuxième conduit distinct du premier conduit et communiquant avec l'embouchure, le détecteur s'étendant dans le deuxième conduit.

4. Inhalateur selon l'une quelconque des revendications 1 à 3, **caractérisé en ce que** l'obturateur (68) est mobile à coulissement dans un canal communiquant avec la chambre, l'orifice (53) étant ménagé dans une paroi latérale du canal, l'obturateur réalisant une liaison étanche avec le canal.

5. Inhalateur selon l'une quelconque des revendications 1 à 4, **caractérisé en ce que** le détecteur comporte un volet rotatif agencé pour être mis en rotation par l'aspiration.

6. Inhalateur selon la revendication 5, **caractérisé en ce que** les moyens d'éloignement comportent un rochet (84) articulé sur l'obturateur, le rochet étant agencé pour être en appui sur le volet (104) lorsque le volet est dans une position de repos, et pour que l'appui soit supprimé lorsque le volet est mis en rotation.

7. Inhalateur selon la revendication 6, **caractérisé en ce qu'**il comporte des moyens de rappel du rochet vers une position d'appui sur le volet.

8. Inhalateur selon la revendication 7, **caractérisé en ce que** les moyens de rappel comportent une lame flexible (100) en appui sur l'obturateur.

9. Inhalateur selon la revendication 8, **caractérisé en ce que** le rochet comporte une paroi (88) présentant un évidement (96), la lame étant reliée à un bord de la paroi et s'étendant en regard de l'évidement.

10. Inhalateur selon l'une quelconque des revendications 6 à 9, **caractérisé en ce que** le rochet comporte un taquet (94) venant en butée contre l'obturateur (68).

11. Inhalateur selon l'une des revendications 6 à 10, **caractérisé en ce que** le rochet comporte au moins une arête vive (115) par laquelle le rochet prend appui sur le volet.

12. Inhalateur selon la revendication 11, **caractérisé en ce que** le volet comporte un tourillon (106) disposé pour servir d'appui à l'arête.

13. Inhalateur selon l'une des revendications précédentes, **caractérisé en ce que** la chambre de compression comporte un piston (30), et l'inhalateur comporte des moyens d'actionnement (41) du piston pour mettre la chambre dans un état comprimé, ces moyens étant agencés pour établir le verrouillage de ces moyens d'actionnement en position comprimée de la chambre.

14. Inhalateur selon la revendication 13, **caractérisé en ce que** les moyens d'actionnement comportent un levier.

## Patentansprüche

1. Pulverinhalator mit einer Luft-Einspeisungskammer (39), einer Leitung, die mit der Kammer durch eine Öffnung (53) in Verbindung steht und in einen Inhalationsstutzen (121) mündet, Mitteln (136, 130), um eine Dosis an Pulver in der Leitung anzuordnen, und einem Fühler (104) für eine Ansaugung, die im Stutzen erzeugt wird, wobei die Kammer eine Kammer für die Kompression von Luft bildet, und wobei der genannte Inhalator außerdem die folgenden Merkmale aufweist:
- ein bewegliches Sperrglied (68), das betrieben wird, um die Öffnung versperren zu können;
- Mittel (84, 104), die durch den Fühler (104) gesteuert sind, um das Sperrglied (68) bezüglich der Öffnung zu entfernen;
- Gasdruckmittel (30) zum Vorbelasten des Sperrgliedes (68) in der Richtung seiner Entfernung von der Öffnung, wenn es die Öffnung sperrt;
- ein Mechanismus (84) zum Festlegen des Sperrgliedes (68) entgegen der Wirkung der Vorbelastungsmittel (30) in einer Lage zum Versperren der Öffnung,
wobei der genannte Inhalator **dadurch gekennzeichnet ist, daß** die Vorbelastungsmittel (30) dazu eingerichtet sind, das Sperrglied (68) in der Absperrlage zu belasten, bevor das Ansaugen durch den Fühler (104) erfaßt wird.

2. Inhalator nach Anspruch 1, **dadurch gekennzeichnet, daß** der Festlegungsmechanismus (84, 104) Teil der Mittel zum Entfernen des Sperrgliedes bildet.

3. Inhalator nach Anspruch 1 oder 2, **dadurch gekennzeichnet, daß** er eine zweite Leitung aufweist, die sich von der ersten Leitung unterscheidet und mit dem Stutzen in Verbindung steht, wobei der Fühler sich in der zweiten Leitung erstreckt.

4. Inhalator nach irgendeinem der Ansprüche 1 bis 3, **dadurch gekennzeichnet, daß** das Sperrglied (68) in einem Kanal gleitbeweglich ist, der mit der Kammer in Verbindung steht, wobei die Öffnung (53) in eine Seitenwand des Kanals eingebracht ist und das Sperrglied eine dichte Verbindung mit dem Kanal herstellt.

5. Inhalator nach irgendeinem der Ansprüche 1 bis 4, **dadurch gekennzeichnet, daß** der Fühler einen drehbaren Flügel aufweist, der dazu eingerichtet ist, um durch die Ansaugung in Drehung versetzt zu werden.

6. Inhalator nach Anspruch 5, **dadurch gekennzeichnet, daß** die Mittel zum Entfernen eine auf dem Sperrglied schwenkbare Klinke (84) aufweisen, wobei die Klinke dazu eingerichtet ist, um auf dem Flügel (104) anzuliegen, wenn sich der Flügel in einer Ruhelage befindet, und um die Anlage aufzuheben, wenn der Flügel in Drehung versetzt wird.

7. Inhalator nach Anspruch 6, **dadurch gekennzeichnet, daß** er Mittel zum Rückstellen der Klinke in eine Lage der Anlage auf dem Flügel aufweist.

8. Inhalator nach Anspruch 7, **dadurch gekennzeichnet, daß** die Rückstellmittel eine flexible Zunge (100) aufweisen, die gegen das Sperrglied anliegt.

9. Inhalator nach Anspruch 8, **dadurch gekennzeichnet, daß** die Klinke eine Wand (88) aufweist, die eine Ausnehmung (96) darbietet, wobei die Zunge mit einem Rand der Wand verbunden ist und sich der Ausnehmung gegenüberliegend erstreckt.

10. Inhalator nach irgendeinem der Ansprüche 6 bis 9, **dadurch gekennzeichnet, daß** die Klinke eine Nase (94) aufweist, die in Anschlag gegen das Sperrglied (68) gelangt.

11. Inhalator nach irgendeinem der Ansprüche 6 bis 10, **dadurch gekennzeichnet, daß** die Klinke mindestens eine scharfe Kante (115) aufweist, mit welcher die Klinke auf dem Flügel aufliegt.

12. Inhalator nach Anspruch 11, **dadurch gekennzeichnet, daß** der Flügel einen Drehzapfen (106) aufweist, der vorgesehen ist, um als Anlage für die Kante zu dienen.

13. Inhalator nach einem der vorangehenden Ansprüche, **dadurch gekennzeichnet, daß** die Druckkammer einen Kolben (30) aufweist, und der Inhalator Betätigungsmittel (41) des Kolbens aufweist, um die Kammer in einen komprimierten Zustand zu versetzen, um die Verriegelung dieser Betätigungsmittel in komprimierter Lage der Kammer herzustellen.

14. Inhalator nach Anspruch 13, **dadurch gekennzeichnet, daß** die Betätigungsmittel einen Hebel aufweisen.

## Claims

1. Powder inhaler including an air supply chamber (39), a conduit communicating with the chamber via an orifice (53) and leading into an inhalation mouthpiece (121), means (136, 130) for bringing a dose of powder into the conduit, and a detector (104) for detecting suction created in the mouthpiece, the chamber constituting an air compression chamber, and the said inhaler additionally including:
- a movable obturator (68) designed to be able to close off the orifice;
- means (84, 104) for distancing the obturator (68) from the orifice, these means being controlled by the detector (104);
- means (30), with compressed gas, for stressing the obturator (68) in the direction away from the orifice when it is closing the orifice;
- a mechanism (84) for immobilizing the obturator (68) counter to the stressing means (30) in a position of closure of the orifice, said inhaler being **characterized in that** the stressing means (30) are able to stress the obturator (68) to the closure position before detection of the suction by the detector (104).

2. Inhaler according to Claim 1, **characterized in that** the immobilizing mechanism (84, 104) forms part of the means for distancing the obturator.

3. Inhaler according to Claim 1 or 2, **characterized in that** it includes a second conduit distinct from the first conduit and communicating with the mouthpiece, the detector extending within the second conduit.

4. Inhaler according to any one of Claims 1 to 3, **characterized in that** the obturator (68) is movable slidably in a channel communicating with the chamber, the orifice (53) being formed in a lateral wall of the channel, the obturator making a leaktight connection with the channel.

5. Inhaler according to any one of Claims 1 to 4, **characterized in that** the detector includes a rotating flap designed to be rotated by the suction.

6. Inhaler according to Claim 5, **characterized in that** the distancing means include a ratchet (84) articulated on the obturator, the ratchet being designed to bear on the flap (104) when the flap is in a rest position, and in such a way that the bearing is suppressed when the flap is set in rotation.

7. Inhaler according to Claim 6, **characterized in that** it includes means for returning the ratchet to a position in which it bears on the flap.

8. Inhaler according to Claim 7, **characterized in that** the return means include a flexible blade (100) bearing on the obturator.

9. Inhaler according to Claim 8, **characterized in that** the ratchet includes a wall (88) having a recess (96), the blade being connected to an edge of the wall and extending in line with the recess.

10. Inhaler according to any one of Claims 6 to 9, **characterized in that** the ratchet includes a catch (94) abutting against the obturator (68).

11. Inhaler according to any one of Claims 6 to 10, **characterized in that** the ratchet includes at least one sharp edge (115) via which the ratchet bears on the flap.

12. Inhaler according to Claim 11, **characterized in that** the flap includes a journal (106) arranged to serve as a bearing for the edge.

13. Inhaler according to one of the preceding claims, **characterized in that** the compression chamber includes a piston (30), and the inhaler includes means (41) for actuating the piston to bring the chamber into a compression state, these means being designed to lock these actuation means when the chamber is in the compression state.

14. Inhaler according to Claim 13, **characterized in that** the actuation means include a lever.
